# EUROPEAN PATENT APPLICATION

(11) **EP 3 189 858 A1**
(43) Date of publication of application: **12.07.2017**
(21) Application number: 14888320.0
(22) Date of filing: 03.04.2014
(51) Int. Cl.: A61L 9/014

(54) **ECOLOGICAL FILTER FOR CONDITIONING AND PRESERVING CORPSES, HUMAN REMAINS AND CADAVEROUS REMAINS**

(71) Applicant: Conserfil Nature S.L., 28038 Madrid (ES)
(72) Inventor: MALAGÓN TRILLO, Felix, E-28038 Madrid (ES); GALACHE RODRIGUEZ, Jose Antonio, E-28038 Madrid (ES)
(74) Representative: Pons Ariño, Angel
(86) International application number: PCT/ES2014/070261
(87) International publication number: WO 2015/150597

(57) **Abstract**

The invention relates to an ecological filter formed by clays and non-vegetable pure active carbons, mixed inside a bag of organic and biodegradable tissue, which adheres to the body, and is used for conditioning and preserving corpses, human remains and cadaverous remains, solving the problems of gases, smells, volatile organic compounds and expulsion of leachates derived from the processes of putrefaction and decomposition of said remains.

## Description

### OBJECT OF THE INVENTION

The present invention relates to a filter formed by natural clays and pure activated carbons, mixed inside a bag made of organic and biodegradable fabric, which is adhered to the body, and which is intended for conditioning and preserving corpses, human remains and cadaverous remains.

The invention is used in the field of handling and transport of the previously defined remains and is intended for solving problems of gases, odours, volatile organic compounds and expulsion of leachates arising from the processes of putrefaction of said remains.

### BACKGROUND OF THE INVENTION

Over the years, techniques have been developed in an attempt to solve the problems arising from the process of putrefaction of human remains, particularly those derived from gases and odours.

The most widely used technique is injecting or introducing chemical substances into the body aimed at slowing down the putrefaction process which are usually toxic, harmful to the operators' health and harmful to the environment. In turn, these techniques involve having to plug the corpses to prevent the expulsion of leachates, in addition to introducing them in cold storage rooms. The present invention does not involve the need to inject any type of toxic substance, since it is a harmless, biodegradable and environmentally friendly device, which additionally does not require excessive handling because it is quickly installed, the corpse does not have to be opened, the pathways of the corpse do not need to be plugged and the remains do not need to be introduced in cold storage rooms.

In turn, other types of techniques have been developed aimed at decreasing the problems of gases and odours, and in some cases fluids, such as patent WO95003769 of Ecoprob BV, which discloses a blanket or mat formed by a series of layers, i.e. a permeable upper layer, an absorbent intermediate layer and a lower waterproof layer, intended mainly to contain and absorb fluids, in the order of 4 l/m², and, likewise, to reduce odour emissions from the incontinence of animals and people to a certain extent, and being usable as a waterproof blanket on beds and seats. While it is true that the intermediate layer contained carbon and zeolite among its many components, these are present in a minority amount and distributed only in one layer, in which the main components are polyethylene, natural fibres, cotton and gel mixtures, wood waste, lignite, hydroexpandable polymers and others. Therefore, it can be considered that this patent differs in the characterisation of the composition, structure and functionality of the invention. Mention can be made of other types of techniques aimed at eliminating gases and odours in closed spaces by means of filters, such as for example patents ES1057857U and ES1053096U of Thomas Higuero, which define two devices for filtering and purifying air by means of an electrofan that generates an air flow which has a series of filters coupled to one its outlets that make it possible to purify the air, in which the use of filters in this purification context is introduced, but that differs from the present invention both in its use, location and composition. Lastly, mention should be made of another patent, ES2400260, filed by the same applicants of the present invention, wherein filters are also used to improve the purification of the space and, specifically, are used to be disposed in containers or receptacles for transporting corpses and, therefore, differ in their method of use, in their physical and chemical characteristics and in their weight% ratio of the components of the present invention, since the filter is placed directly on the corpse, requiring a specific composition detailed below and, since it is placed directly on the skin, requires containment and fixing means, as opposed to earlier techniques that are not applied directly to the application area, but rather to an open area, corpse container or similar receptacle.

With the present invention a wide range of improvements and innovations over earlier techniques are achieved, mainly that the filter is placed directly on the skin or body of the corpse; it is quick and easy to handle, at room temperature, without need to be inside a hospital or morgue so that the body does not degrade, since the body does not have to be opened or sutured, does not require the use of cold storage rooms, nor do the body's pathways have to be plugged; there is no expulsion of leachates or bad odour; a manageable and transportable product is achieved that does not require technical expertise to be installed; a product is achieved that extends preservation time, since the greater the number of filters are placed on the corpse, the longer said time is extended; a harmless, biodegradable product not harmful to humans or to the environment is achieved; a non-aggressive product is achieved that ensures that neither the pigmentation of the body nor the body's pH varies; it can be used at any time after death; it stops the generation of bacteria and halts the destruction of cells by means of a non-toxic compound; in addition to the relevant economic improvement in production. Lastly, it can be said that the invention focuses on demonstrating that this new technique significantly improves earlier techniques and, in fact, could be used in combination with any of the foregoing.

### DESCRIPTION OF THE INVENTION

The present invention described herein is an ecological filter that is placed directly on the skin of the corpse, human remains or cadaverous remains, which is composed of a bag made of organic, porous and biodegradable fabric, preferably absorbent polypropylene, which houses a mixture of compounds in its interior that make up the filter itself, said bag being closed, hermetically, by heat-sealing, and the filter adhered to the corpse by means of a fixing element such as adhesive tape, adherent means or similar adhesive elements.

As mentioned, the filter is adhered or attached to the skin of the corpse and is preferably placed on the iliac fossae and on the upper and lower grids, although it can be located on any part of the body in which the decomposition process is more advanced.

The whole is secured by means of adhesive tape or similar to the corpse so that, in case of movement of the coffin or similar mortuary container, the filter does not fall off or move from the desired place.

The mixture housed inside the bag is composed of non-vegetable pure activated carbons and natural clays sprinkled with potassium permanganate, and wherein the functionality of the whole is based on the principle of adsorption.

The clays, which are natural clays, are composed of a mixture of zeolites, sepiolites, bicarbonates and other deodorising clays such as kaolinite and/or bentonite. An oxidising agent is sprinkled over these clays, preferably potassium permanganate, in a very low percentage, which, mixed and once saturated, is transformed into manganese dioxide. The mixture of natural clays absorbs gases, odours and volatile organic compounds. Inside the bag, the clay mixture is in the form of 3 mm pellets.

Pure activated carbons, which are non-vegetable carbons, act against leachate generation. Inside the bag, these carbons are in the form of 4 mm pellets.

The mixture of the components, dry mixed, with the ranges (as a weight%) or recommended margins, combinable such as to always total 100% and without rejections, the following:
- Clays 69.50% - 90.30%
- Pure activated carbons 9.70% - 31.50%

Given that the ranges (as a weight%) or recommended margins of the clay mixture, combinable such as to always total 100% without rejections, the following:
- Natural clays composed of zeolites, sepiolites and bicarbonates 88.10% - 97.50%
- Potassium permanganate 2.50% - 11.90%

Wherein the permanganate is dry-sprinkled over the natural clay mixture.

And wherein lastly, the relationship between the natural clay mixture (as a weight%) or recommended margins, combinable such as to always total 100% without rejections, the following:
- Zeolite 30.00% - 85.00%
- Sepiolite 30.00% - 85.00%
- Bicarbonates 10.00% - 40.00%
- Other kaolinite- and bentonite-type deodorising clays 0.10% - 10.00%

The bags containing these mixtures may be of varying sizes and shapes, preferably 150 g to 900 g to facilitate handling and transport, and are ready to be placed on the skin of the corpse at any time after death and in any place where said body is located, since it is not necessary to be inside a hospital or morgue.

Lastly, it should be noted that, since these filters are placed directly on the skin of the corpse, said corpse may be dressed in clothing or a shroud without being noticed from the outside.

### Example of preparation

400 g of mixture are introduced into a bag made of absorbent polypropylene, specifically 335 g of clay in 3 mm pellets and 65 g of non-vegetable activated carbon in 4 mm pellets are introduced; considering that 25 g of potassium permanganate has been impregnated in 310 g of the mixture of natural clays, which are dry mixed, and the mixture of these natural clays is composed of 130 g of zeolite, 130 g of sepiolite, 45 g of bicarbonate and 5 g of the bentonite and kaolinite mixture. Once these components have been introduced into the bag is closed by means of heat-sealing, and is fixed to the corpse by means of adhesive tapes.

Having sufficiently described the nature of the invention in the foregoing, taking into account that the terms drafted in this specification should be taken in a broad and non-limiting sense, in addition to the description of the manner in which to put it into practice, and having demonstrated that it constitutes a positive technical advance, on the basis of which this patent application is filed, being what constitutes the essence of the subject invention that specified in the following claims.

## Claims

1. An ecological filter for conditioning and preserving corpses, human remains and cadaverous remains, **characterised in that** 9.70% - 31.50% (by weight) of non-vegetable pure activated carbons are mixed with 69.50% - 90.30% (by weight) of clays composed of natural clays sprinkled with potassium permanganate, independently adjusted so as to total 100%, both components being in the form of pellets, inside a bag made of organic, porous and biodegradable fabric, which is closed by heat-sealing and adhered directly to the skin of a corpse by fixing means.

2. The ecological filter for conditioning and preserving corpses, human remains and cadaverous remains, according to claim 1, **characterised in that** the mixture of clays is dry mixed and is formed by 88.10% - 96.50% (by weight) of natural clays and 3.50% - 11.90% (by weight) of potassium permanganate, independently adjusted so as to total 100%.

3. The ecological filter for conditioning and preserving corpses, human remains and cadaverous remains, according to claims 1 and 2, **characterised in that** the mixture of natural clays is dry mixed and is formed by 30.00% - 85.00% (by weight) of zeolites, 30.00% - 85.00% (by weight) of sepiolites, 10.00% - 40.00% (by weight) of bicarbonates and 0.10% - 10.00% (by weight) of other kaolinite- and bentonite-type deodorising clays, independently adjusted so as to total 100%.

4. The ecological filter for conditioning and preserving corpses, human remains and cadaverous remains, according to claim 1, **characterised in that** the bag is made of absorbent polypropylene.

5. The ecological filter for conditioning and preserving corpses, human remains and cadaverous remains, according to claim 1, **characterised in that** the fixing means used to fix the bag to the skin of the corpse is adhesive tape.
